# EUROPEAN PATENT APPLICATION

(11) **EP 3 660 160 A1**
(43) Date of publication of application: **03.06.2020**
(21) Application number: 18382863.1
(22) Date of filing: 28.11.2018
(51) Int. Cl.: C12Q 1/04, C12M 1/34, G01N 33/543

(54) **PORTABLE DEVICE AND METHOD FOR DETECTING MICROORGANISMS OR METABOLITES IN A SAMPLE**

(71) Applicant: GTZ Microlab Detect, S.L., 48170 Zamudio - Bizkaia (ES)
(72) Inventor: FONT OJEDA, Armando, 50014 ZARAGOZA (ES); RAZQUIN CASQUERO, Pedro, 50012 ZARAGOZA (ES); MATA VALLESPIN, Luis, 50012 ZARAGOZA (ES)
(74) Representative: Ponti & Partners, S.L.P

(57) **Abstract**

The present invention relates to a portable device and a method for detecting microorganisms or metabolites in a sample (S), the device comprising:
- an enrichment chamber (14) containing a culture medium (M), closed by a first tearable seal (13),
- a collecting member for collecting a sample (S);
- means for tearing the first tearable seal (13) to make the sample (S) enter into the enrichment chamber (14);
- a mixture chamber (5m) comprising a sealed, but openable, inlet (5ma), to receive a portion of an enriched mixture from the enrichment chamber (14);
- a detection chamber (5d) to receive and detect an aliquot of the portion of the enriched mixture;
- an opening member for opening the sealed inlet (5ma); and
- a fluidic communication member for communicating the detection chamber (5d) with the mixture chamber (5m) only once the inlet (5ma) thereof is opened.

## Description

### FIELD OF THE INVENTION

The present invention generally relates, in a first aspect, to a portable device for detecting microorganisms or metabolites in a sample, and more particularly to a portable device which constitutes a kind of microbiologic laboratory for in situ detection without the need of qualified personnel, that allows to perform all the phases needed for the detection within the device in a safe manner.

A second aspect of the invention relates to a method for detecting microorganisms or metabolites in a sample by using the portable device of the first aspect.

### BACKGROUND OF THE INVENTION

Some portable devices for detecting microorganisms or metabolites in a sample are known in the art.

Particularly, EP2483388A2 discloses one of those portable devices, and comprises all the features of the preamble of claim 1 of the present invention, i.e.:
- an enrichment chamber for containing a culture medium in a sealed manner, wherein a mouth of said enrichment chamber is closed by a first tearable seal,
- a collecting member for collecting a sample by its deposition on a supporting wall thereof;
- means for tearing said first tearable seal to provide or allow the displacing of said sample from said supporting wall to the interior of said enrichment chamber through the torn first tearable seal, so that the sample is enriched with said culture medium for a microorganism or metabolite of interest, and an enriched mixture of said culture medium and said sample is provided;
- a mixture chamber fluidically communicable with the enrichment chamber for receiving a portion of said enriched mixture from the enrichment chamber; and
- a detection chamber fluidically communicable with said mixture chamber to receive at least an aliquot of said portion of the enriched mixture therefrom, said detection chamber housing a detection element for detecting the presence of said microorganism or metabolite of interest in said aliquot.

The mixture chamber of the portable device disclosed in said patent application is always fluidically communicated with the enrichment chamber, even when the culture medium is not yet in contact with the sample, not being therefore possible to select at which moment to allow the reception of the enriched mixture therein, such as only once the enriched mixture is provided.

Also, the detection chamber of the portable device disclosed in EP2483388A2, which is generally part of an external device (such as an injector) can be communicated with the mixture chamber at any moment, with no constraint, no matter if the enrichment mixture has already been received into the mixture chamber.

It is therefore necessary to provide an alternative to the state of the art which covers the gaps found therein, by providing a portable device for detecting microorganisms or metabolites in a sample, having improved features in order to allow an even safer and easier in situ detection without the need of qualified personnel.

### SUMMARY OF THE INVENTION

To that end, the present invention relates, in an aspect, to a portable device for detecting microorganisms or metabolites in a sample, comprising:
- an enrichment chamber for containing a culture medium in a sealed manner, wherein a mouth of said enrichment chamber is closed by a first tearable seal,
- a collecting member for collecting a sample (solid or liquid) by its deposition on a supporting wall thereof;
- means for tearing said first tearable seal to provide or allow the displacing of said sample from said supporting wall to the interior of said enrichment chamber through the torn first tearable seal, so that the sample is enriched with said culture medium for a microorganism or metabolite of interest (such as salmonella, listeria, etc.), and an enriched mixture of said culture medium and said sample is provided;
- a mixture chamber fluidically communicable with the enrichment chamber for receiving a portion of said enriched mixture from the enrichment chamber; and
- a detection chamber fluidically communicable with said mixture chamber to receive at least an aliquot of said portion of the enriched mixture therefrom, said detection chamber housing a detection element for detecting the presence of said microorganism or metabolite of interest in said aliquot.

In contrast to the portable devices known in the art, in the one provided by the first aspect of the present invention, the mixture chamber comprises a sealed, but openable, inlet, to receive the portion of the enriched mixture from the enrichment chamber flowing through the torn first tearable seal and then through said inlet, once opened, and the portable device further comprises:
- an opening member for opening said sealed inlet of the mixture chamber; and
- a fluidic communication member configured and arranged for fluidically communicating the detection chamber with the mixture chamber only once the sealed inlet of the mixture chamber is already opened.

For an embodiment, preferably intended for a case for which the sample is a solid sample, the portable device further comprises a filter arranged between the enrichment chamber and the mixture chamber to filter the portion of the enriched mixture to be received by the mixture chamber.

Preferably, the above mentioned supporting wall and first tearable seal are the same element, so that when the first tearable seal is torn by the above mentioned means and the portable device is in an upright position, at which the mouth of the enrichment chamber is in an upper position, the sample being supported thereon is automatically displaced by falling by gravity into the enrichment chamber.

Alternatively, for less preferred embodiments, the supporting wall and the first tearable seal are different elements, so that when the first tearable seal is torn the sample supported on the supporting wall is displaced towards the enrichment chamber through the torn first tearable seal, whether only by gravity (for example, if the supporting wall is highly inclined with respect to the first tearable seal) and/or by exerting an external force thereon (by pushing, or just by shaking the portable device).

For a preferred embodiment, the above mentioned opening member and fluidic communication member are the same element, said same element being a moving member configured and arranged to displace according to at least a first displacement path, to adopt a first position at which it opens the sealed inlet of the mixture chamber and, only once said first position has been adopted, move to adopt a distinct and second position at which it fluidically communicates the detection chamber with the mixture chamber.

For an implementation of said preferred embodiment, the detection chamber is communicated with the mixture chamber through at least one fluidic channel, and comprises a sealed, but openable, aperture configured and arranged to be opened by the moving member when at said second position, so that pressure within the detection chamber equals that of the mixture chamber, the mixture and the detection chambers thus forming communicating vessels so that said aliquot of the portion of the enriched mixture enters into the detection chamber from the mixture chamber and passing through the at least one fluidic channel.

For an embodiment, the inlet of the mixture chamber is sealed by a tearable seal, and said moving member has a tearing portion configured and arranged to tear said tearable seal at least when at said first position.

For a further embodiment, the aperture of the detection chamber is also sealed by a tearable seal, and the tearing portion of the moving member is configured and arranged to tear said tearable seal of the aperture of the detection chamber at least when at said distinct and second position.

For an embodiment, a common tearing seal is used for both the inlet of the mixture chamber and the aperture of the detection chamber, the above mentioned tearable seals thereof being different regions of said common tearable seal.

Alternatively, the tearable seals of the inlet of the mixture chamber and the aperture of the detection chamber are independent tearing seals.

Advantageously, the moving element is configured and arranged to tear, by means of the tearing portion thereof:
- the tearable seal of the inlet of the mixture chamber along a plurality of positions, including the above mentioned first position, that are adopted through the displacement of the moving member according to at least the first displacement path; and/or
- the tearable seal of the aperture of the detection chamber along a plurality of positions, including the above mentioned second position, that are adopted through the displacement of the moving member according to at least said first displacement path.

According to an embodiment, the moving member is configured and arranged to displace also according to a second displacement path that is transversal to a plane occupied by the tearable seals of the inlet of the mixture chamber and of the aperture of the detection chamber, so that during the displacement of the moving member along the first displacement path the tearing portion moves according to said second displacement path towards the mixture chamber or the detection chamber when facing the corresponding tearable seal.

For an embodiment, the moving member is configured and arranged so that during said displacement according to said second displacement path the tearing portion:
- progressively penetrates into the mixture chamber, through the corresponding tearable seal, increasingly until the first position is achieved along the displacement according to the first displacement path, and decreasingly therefrom, and then
- progressively penetrates into the detection chamber, through the corresponding tearable seal, increasingly until the second position is achieved along the displacement according to the first displacement path, and decreasingly therefrom.

When the portable device of the first aspect of the present invention is in an upright position, for an embodiment, the mixture and detection chambers project upwards from respective adjacent locations of a base wall, wherein said adjacent locations follow a first circumferential path over the first tearable seal.

According to an embodiment, the moving member is located between the first tearable seal and the mixture and detection chambers and is articulated to rotate about a first articulation axis that is orthogonal to a plane occupied by said first tearable seal, to displace according to the first displacement path, which is a rotation displacement path.

For an alternative embodiment, the first displacement path is a linear displacement path.

The portable device of the first aspect of the present invention comprises, for an embodiment, a manual rotary actuator mechanically connected to the moving member, and that is accessible to a user (for example by means of a rotary knob) to rotate the moving member according to the first, rotating, displacement path. Alternatively, an automatic rotary actuator is arranged instead of said manual rotary actuator.

According to an embodiment, the moving member is further articulated about a second articulation axis that is orthogonal to the first axis and pivots there about, to displace according to the second displacement path, which is a pivoting displacement path.

For an implementation of said embodiment, the tearing portion is located at a first end of the moving member, the moving member having a second end, opposite to said first end, that is guided by means of ramps, defined by adjacent extensions projecting towards the first tearable seal from said base wall and following a second circumferential path, to make the moving member pivot about said second articulation axis when being displaced according to the first, rotating, displacement path, to provide the above mentioned progressive penetration of the tearing portion into the mixture and the detection chambers.

According to a further embodiment, the portable device of the first aspect of the present invention further comprising at least one of:
- a reactive chamber for containing a reactive agent, that is fluidically communicable with the enrichment chamber through a sealed, but openable, aperture, for providing said reactive agent thereto through said torn first tearable seal, wherein said or another opening member is configured and arranged to open said sealed aperture of said reactive chamber before the opening of the sealed inlet of the mixture chamber; and
- an inactivating chamber containing an inactivating agent, that is fluidically communicable with the enrichment chamber through a sealed, but openable, aperture, for providing said inactivating agent thereto through said torn first tearable seal, wherein said or another opening member is configured and arranged to open said sealed aperture of said inactivating chamber only after the detection chamber has been fluidically communicated with the mixture chamber.

For an implementation of said embodiment, the moving member is configured and arranged to move to tear, with its tearing portion, respective seals of the aperture or inlets of the different chambers according to the following order: first the reactive chamber, then the mixture chamber, then the detection chamber, and finally the inactivating chamber,

The present invention also relates, in a second aspect, to a method for detecting microorganisms or metabolites in a sample, using a portable device according to any of the previous claims, the method comprising sequentially performing the following steps:
- providing the enrichment chamber filled with a culture medium in a sealed manner,
- depositing a sample on said supporting wall of the collecting member;
- tearing the first tearable seal and displacing the sample from the supporting wall to the interior of the enrichment chamber through the torn first tearable seal,
- incubating the contents of the enrichment chamber to provide an enriched mixture of said culture medium and said sample;
- opening the sealed inlet of the mixture chamber and providing thereto a portion of said enriched mixture from the enrichment chamber through the torn first tearable seal and the opened inlet;
- fluidically communicating the detection chamber with the mixture chamber to receive at least an aliquot of the portion of the enriched mixture therefrom; and
- detecting the presence of said microorganism or metabolite of interest in said aliquot, by means of said detection element.

### BRIEF DESCRIPTION OF THE FIGURES

In the following some preferred embodiments of the invention will be described with reference to the enclosed figures. They are provided only for illustration purposes without however limiting the scope of the invention.
Figure 1 is an exploded perspective view of the portable device of the first aspect of the present invention, for an embodiment;
Figure 2 is a perspective view of the portable device of Figure 1, with its components mounted, partially sectioned to show the interior of the mixture and detection chambers;
Figure 3 is a cross-section side view of the device of Figure 2 taken through a cut plane running vertically through the centre of the device;
Figure 4 shows part of the device of Figure 3, in a perspective and cross-sectioned view, in a situation where the tearing portion of the moving member is tearing a tearable seal of one of the chambers of the device;
Figure 5 is a cross-sectioned view corresponding to the one of Figure 3 but where two parts of the device are shown decoupled, particularly a bottom part including the enrichment sealed chamberfilled with a culture medium and with a solid sample deposited on the first tearing seal, and an upper part with the components shown in Figure 4;
Figure 6 is a cross-section side view analogous to that of Figure 3 but showing the operation of the stop elastic member to unlock the screwing clockwise of the upper part on the lower part of the device as shown in Figure 5 according to a coupling course;
Figure 7 is analogous to that of Figure 7, but showing a coupling position of the upper part along the coupling course that is reached after overcoming the stop elastic member, so that the first tearable seal is torn by cutting elements arranged on the upper part;
Figure 8 is a perspective view that shows the same device as in Figure 7 and for the same coupling position, showing how the stop elastic member also blocks the unscrewing displacement of the upper part so to prevent the uncoupling of both parts; and
Figure 9 is a side view and a top view of the portable device of the present invention, for the embodiment illustrated in the rest of Figures, together with an enlarged detail of some components thereof; the device is shown with both parts coupled at a maximum coupling course position.

### DESCRIPTION OF PREFERRED EMBODIMENTS

The appended Figures show a preferred embodiment of the portable device of the first aspect of the present invention.

For that preferred embodiment, the device comprises a lower part and an upper part coupleable to each other.

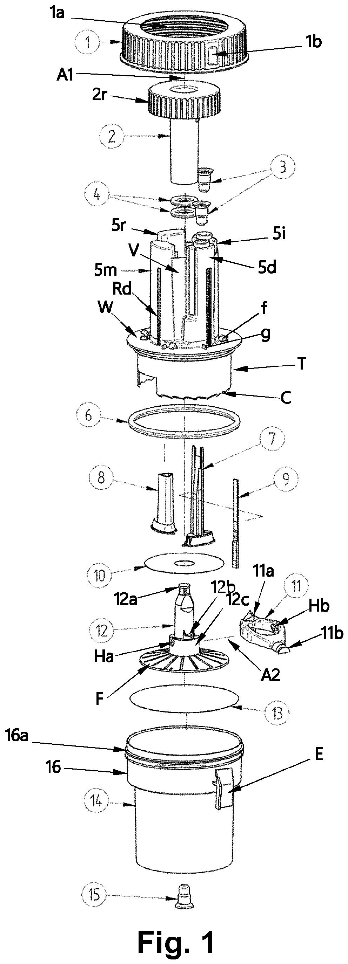

The lower part comprises, as shown in Figures 1, and 3 to 7:
- an enrichment chamber 14, or container, for containing a culture medium M in a sealed manner, wherein a mouth of the enrichment chamber 14 is closed by a first tearable seal 13, and
- a collecting member, particularly a collecting chamber 16, for collecting a sample S by its deposition on a supporting wall thereof, wherein, for the illustrated embodiment, the supporting wall is the first tearable seal 13.

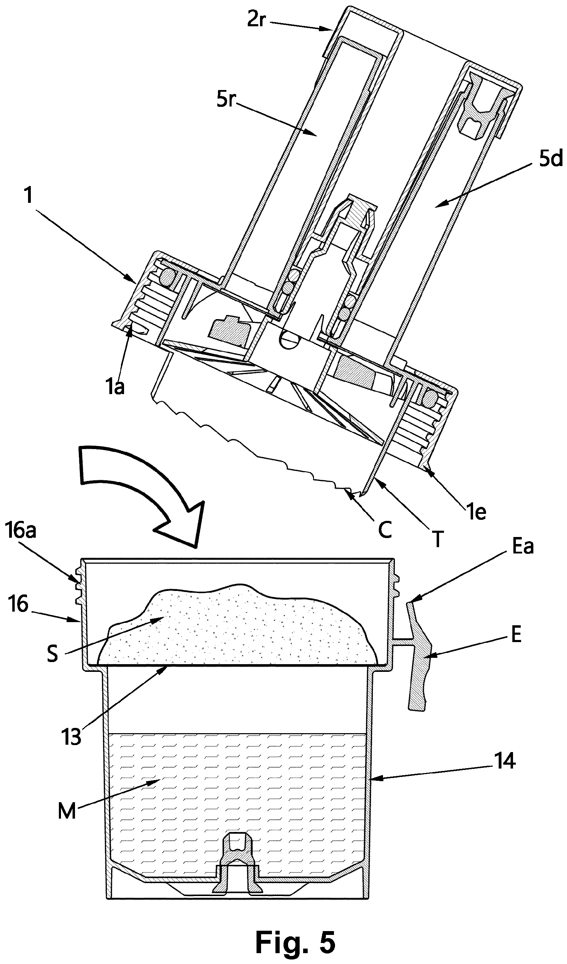

Figure 5 shows the upper and lower parts uncoupled, with a sample S (in this case a solid sample) having been introduced within the collecting chamber 16 deposited on the first tearable seal 13.

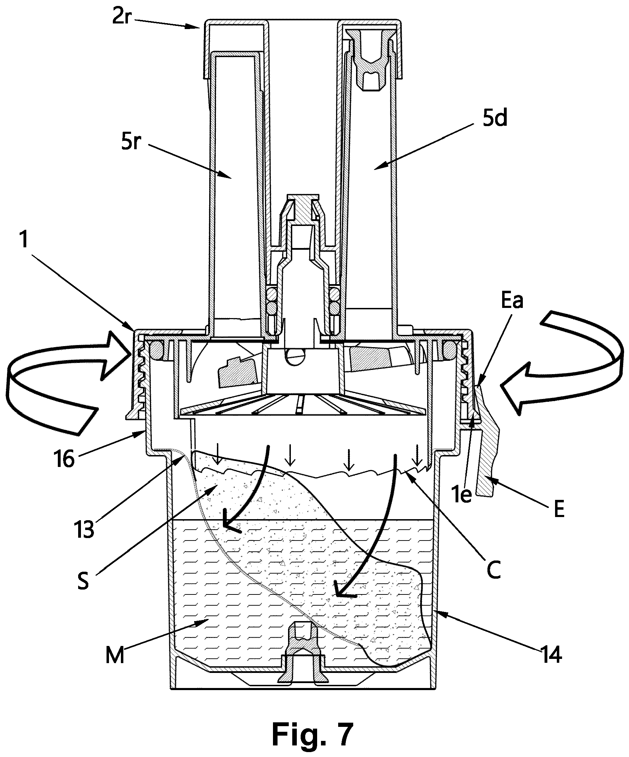

The upper part of the device comprises, as shown especially in Figures 1, and 3 to 7, means for tearing the first tearable seal 13, so that both the collecting chamber 16 and the enrichment chamber 14 are communicated such that the sample S falls into the interior of the enrichment chamber 14 (as shown in Figure 7) through the torn first tearable seal 13, so that the sample S is enriched with the culture medium M for a microorganism or metabolite of interest, and an enriched mixture of the culture medium M and the sample S is provided. For the illustrated embodiment, those means for tearing comprise cutting elements C arranged on the peripheral free edge of a hollow tubular portion T projecting downwards (according to the spatial position shown in Figure 1) from a circular base wall W.

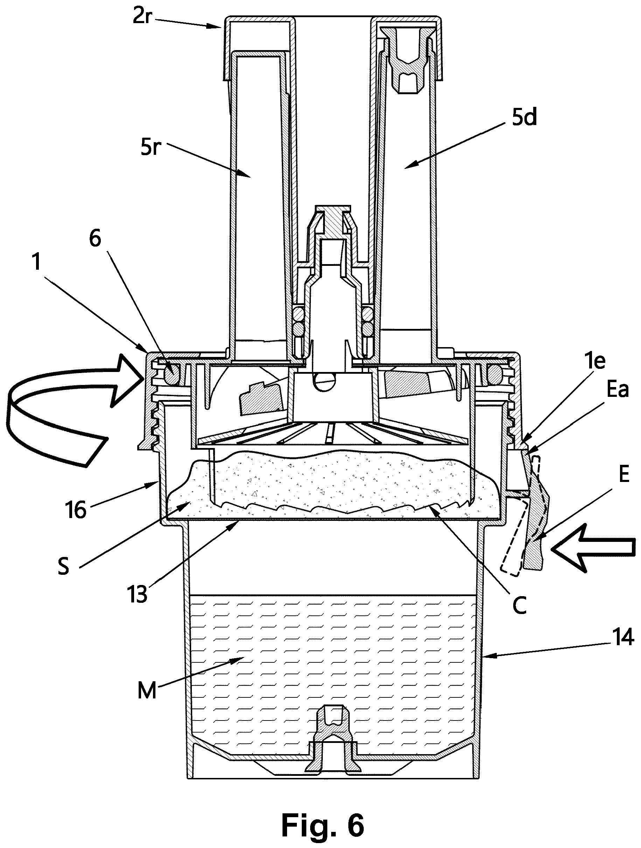

The operation of the above mentioned means for tearing is shown in Figures 6 to 7.

Particularly, Figure 6 shows how the upper part is coupled to the lower part by screwing, particularly by means of the two mutually threaded complementary surfaces 1a, 16a (see Figures 1 and 5) respectively defined in an inner surface of a ring part 1 and an outer surface of the collecting chamber 16.

As shown in Figure 6, at the illustrated coupling course position, the screwing displacement (in this case clockwise) is blocked when the free circumferential edge 1e of the ring part 1 abuts against the upper edge Ea of a stop elastic member E, so that the cutting elements can't reach the tearable seal 13, which remains untorn, thus avoiding an accidental tearing thereof.

In order to unlock said screwing displacement, to cause the tearing of the tearable seal 13, the stop elastic member E must be pressed by a user as indicated by the horizontal arrow depicted in Figure 6, until achieving the position at which the stop elastic member is illustrated in a dashed line in Figure 6.

Then, as illustrated in Figure 7, the screwing displacement can go on so that the ring part 1 (and thus the components attached thereto) goes downwards, the cutting elements C reach and tear the tearable seal 13 such that the sample S falls by gravity into the enrichment chamber 14, and a maximum coupling course position is reached, providing a stable coupling and also in a sealed manner thanks to the sealing ring 6 being trapped against the inner wall of the collecting chamber 16.

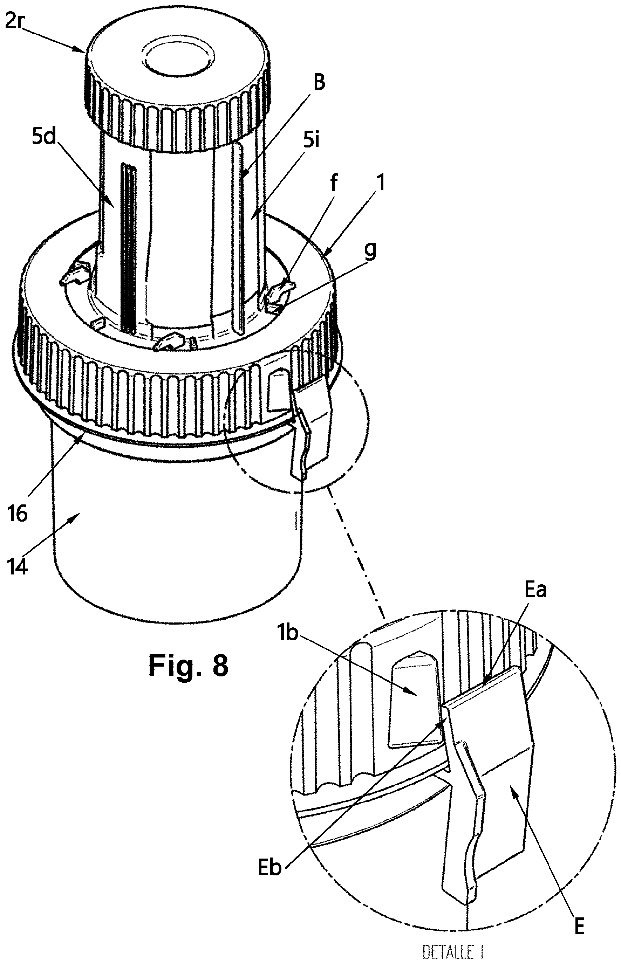

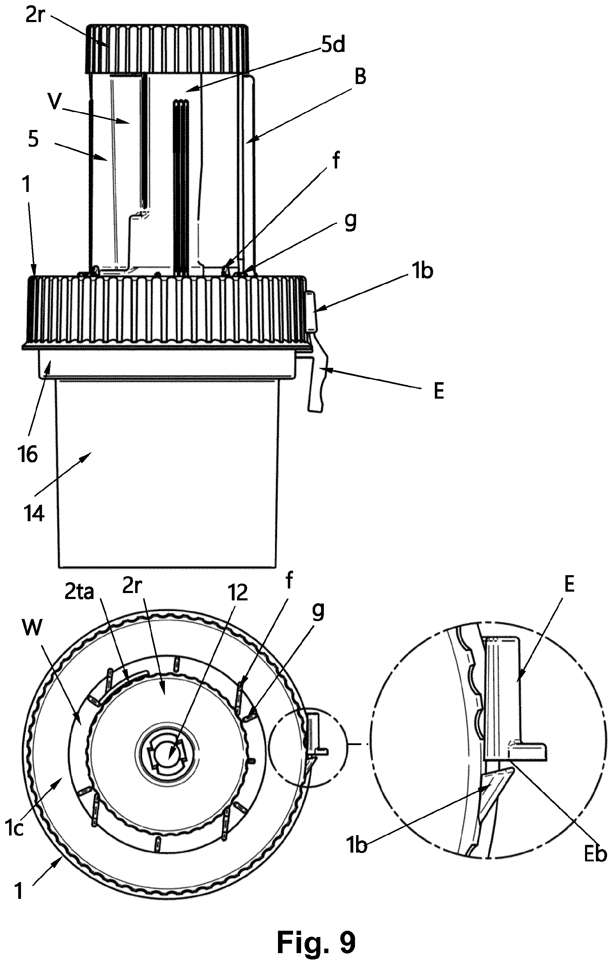

To avoid a possible leak of the contents of the enrichment chamber 14, besides the action of the above mentioned sealing ring 6, the full unscrewing of the ring part 1 is blocked. That's carried out, for the illustrated embodiment, also by means of the stop elastic member E, particularly, as shown in Figures 8 and 9, by means if its side Eb and projection 1b that protrudes from an outer side surface of ring part 1, which collide against each other when only a part of a turn of an unscrewing displacement of the ring part 1 is carried out.

As shown especially in Figures 1, 2, 4 and 9, the portable device of the first aspect of the present invention further comprises a mixture chamber 5m that comprises a sealed, but openable, inlet 5ma, to be fluidically communicable with the enrichment chamber 14 for receiving a portion of the enriched mixture from the enrichment chamber 14, once a seal 10 sealing that inlet 5ma is torn.

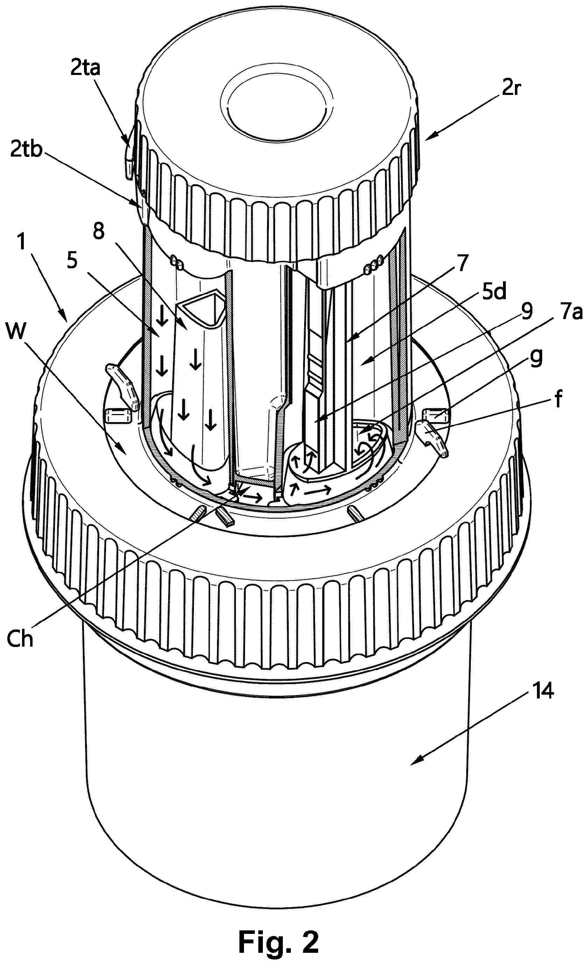

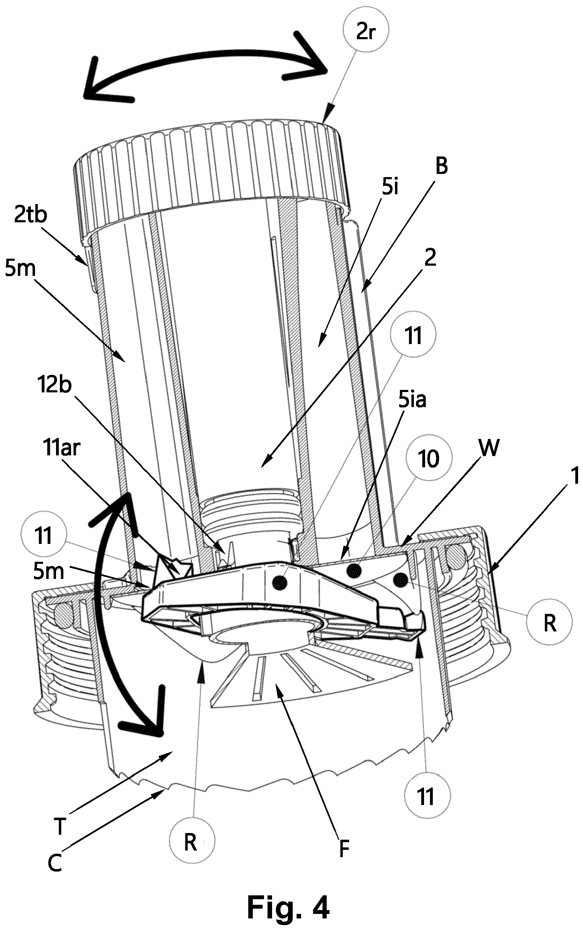

Also for the illustrated embodiment, particularly as shown in Figures 1 and 2, a removable tubular member 8 is placed within the mixture chamber 5m (not shown in Figure 4, for clarity sake), that tubular member 8 being fluidically communicated with the inlet 5ma, so that the portion of the enriched mixture is received into the mixture chamber by flowing through the tubular member 8 and outing through the upper end of the tubular member 8. For that enrichment mixture reception to happen, the portable device must be upside down with respect to the upright position illustrated in the Figures, or at least facing down, if not vertically in an inclined manner with an inclination that is enough to make the portion of the enrichment mixture flow into the mixture chamber 5m through the tubular member 8.

For another embodiment (non-illustrated), tubular member 8 could be non-removable but fixedly housed within the mixture chamber 5m.

Once that's happened, the portable device must be put back into the upright position, so that the portion of the enrichment mixture occupies an inner volume of the mixture chamber 5m adjacent to the tubular member 8, and remains therein until is communicated with an adjacent detection chamber 5d that will be described below.

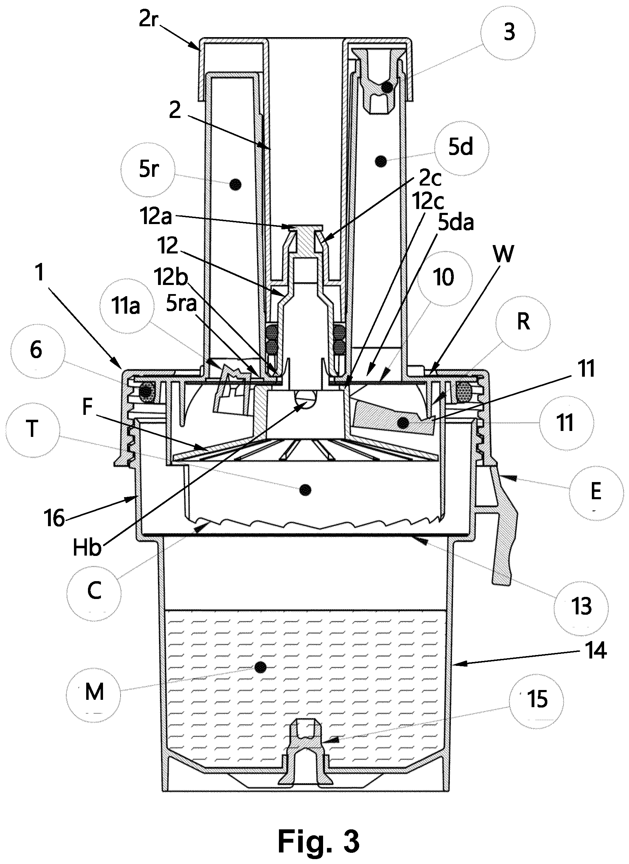

As shown also in Figures 1, 2, 3, and 5 to 9, the portable device also comprises the above mentioned detection chamber 5d, which is fluidically communicable with the mixture chamber 5m to receive an aliquot of the portion of the enriched mixture therefrom, so that the presence of the microorganism or metabolite of interest can be detected in the aliquot by means of a detection element 9 (in this case a chromatographic strip) housed there within. Particularly, as shown in Figure 2, the detection chamber 5d is communicated with the mixture chamber 5m through a fluidic channel Ch, and comprises a sealed, but openable, aperture 5da (see also Figure 3) configured and arranged to be opened so that pressure within the detection chamber 5d equals that of the mixture chamber 5m, the mixture 5m and the detection 5d chambers thus forming communicating vessels so that the aliquot of the portion of the enriched mixture enters into the detection chamber 5d from the mixture chamber 5m, particularly from the above mentioned volume adjacent to the tubular member 8, and passing through the fluidic channel Ch, as signed by the arrow lines depicted in Figure 2.

Preferably at least a portion of the detection chamber 5d is translucent at a region through which the detection element can be seen from outside the device, so that, in case of a chromatographic strip, the results of the detection can be seen by looking thereto through said translucent region.

For the illustrated embodiment, a common tearable seal 10 is used for sealing both the inlet 5ma of the mixture chamber 5m and the aperture 5da of the detection chamber 5d, so that different regions thereof seal the corresponding inlet 5ma and aperture 5da.

It must be highlighted that the regions of the common tearable seal 10 sealing aperture 5da cannot be torn if inlet 5ma has not yet been torn, in order to avoid a malfunctioning of the device. That's achieved by a mechanism that will be described further below.

As shown in Figures 1 and 2, for the illustrated embodiment, the detection element 9 is supported on a support member 7 removably placed within the detection chamber 5d (for a non-illustrated embodiment, that support member 7 could instead be fixedly attached within the detection chamber 5d). That support member 7, as shown in Figure 2, is opened at a bottom portion adjacent to the detection element 9, by means of a through opening 7a fluidically communicated with aperture 5da, for the purpose of draining back to the enrichment chamber 14 an excess of the portion of the enrichment mixture received there into (as signed by the arrow lines depicted in Figure 2), such that only a predetermined aliquot thereof remains within the detection chamber 5d, in this case in contact with the lower part of the detection element 9.

For the tearing of those regions of the common tearable seal 10 (for a non-illustrated embodiment two independent seals could be used instead of a common seal), for the illustrated embodiment, a moving member 11 is provided that is configured and arranged to displace according to at least a first displacement path, to adopt a first position at which a tearing portion 11a thereof tears the region of the common tearable seal 10 sealing inlet 5ma of the mixture chamber 5m (as showing in Figure 4) and, only once that first position has been adopted, move to adopt a distinct and second position at which the tearing portion 11a tears the region of the common tearable seal 10 sealing aperture 5da of the detection chamber 5d, so that the aliquot of the portion of the enriched mixture enters into the detection chamber 5d as explained above, coming from the mixture chamber 5m through channel Ch.

As shown in Figure 1, for the illustrated embodiment, the moving member 11 is located between the first tearable seal 13 and the mixture 5m and detection 5d chambers and is articulated to rotate about a first articulation axis A1 (in this case, a vertical axis) that is orthogonal to a plane occupied by the first tearable seal 13, to displace according to the first displacement path, which is a rotation displacement path.

A manual rotary actuator 2 mechanically connected to the moving member 11 is provided for the illustrated embodiment, and that is accessible to a user, in this case through a rotary knob 2r, to rotate the moving member 11 according to the first, rotating, displacement path. For the illustrated embodiment, that rotation displacement is carried out counterclockwise, so that the region of the common seal 10 sealing inlet 5ma is torn before the one sealing aperture 5da: however, if, for a non-illustrated embodiment, the position of the mixture 5m and detection 5d chambers was interchanged, then the device would be modified so that that rotation displacement would be clockwise.

The moving member 11 is configured and arranged to displace also according to a second displacement path that is transversal to a plane occupied by the tearable seal 10, so that during the displacement of the moving member 11 along the first, rotation, displacement path the tearing portion 11a moves according to the second displacement path towards the mixture chamber 5m or the detection chamber 5d when facing the corresponding region of the tearable seal 10.

That displacement along the second displacement path is provided by means of the moving member 11 being further articulated about a second articulation axis A2 (see Figure 1) that is orthogonal to the first axis A1 and pivots there about, to displace according to the second displacement path, which is a pivoting displacement path.

As shown, among others, in Figures 1, 3 and 4, the tearing portion 11a is located at a first end of the moving member 11, the moving member 11 having a second end 11b, opposite to the first end 11a, the moving member being articulated about the second articulation axis A2 at an intermediate point such that the tearing portion 11a and the second end 11b go in opposite directions when the second displacement occurs.

As shown in Figure 1, the moving member 11 is specifically articulated to a further moving member 12, by means of articulation configurations Hb, Ha, to cause the pivoting displacement about the second axis A2.

The second end 11b is guided by means of ramps defined by adjacent extensions R projecting towards the first tearable seal 13 from the base wall W and following a second circumferential path having a larger diameter than the first circumferential path, to make the moving member 11 pivot about the second articulation axis A2 when being displaced according to the first, rotating, displacement path.

The extensions R have a triangular-like shape formed by two respective ramps converging at a lower convergence point, and are arranged so that when and during the displacement of the moving member 11 rotating about the first axis A1, the second end 11b contacts and runs first through a first ramp of a respective extension R such that that second end 11b is pushed downwards (according to the illustrated position) until it reaches the convergence point, and hence, by pivoting the moving member 11 about the second articulation axis A2, as shown in Figure 4, the first end including the tearing portion 11a is pushed upwards so that it progressively tears the corresponding region of the tearable seal 10 covering inlet 5ma and penetrates into the mixture chamber 5m there through, increasingly until the above mentioned first position is achieved along the rotation displacement about the first articulation axis A1.

Once the second end 11b is at said convergence point, when continuing with the rotation displacement of the moving member 11 in the same direction (counterclockwise for the illustrated embodiment), the tearing portion 11a continues the progressive tearing of the rest of the corresponding region of the tearable seal 10 according to a decreasing penetration caused by the tearing portion 11a being pushed away from the mixture chamber 5m, so that, by pivoting the moving member 11 about the second articulation axis A2, the second end 11b is pushed towards the base wall W running through the other ramp of the extension R.

The above mentioned pushing away from the mixture chamber 5m of the tearing portion 11a is caused by the running through a ramp 11ar thereof of a portion of the contour of the inlet 5mi.

When continuing with the rotation displacement of the moving member 11, the same process is repeated for the progressive tearing of the region of the common seal 10 covering the aperture 5da of the detection chamber 5d, in this case with the second end 11b running through the ramps of another extension R.

For the illustrated embodiment, the mixture 5m and detection 5d chambers project upwards, when the portable device is in an upright position, from respective adjacent locations of a base wall W, wherein said adjacent locations follow a first circumferential path over said first tearable seal 13,

For the illustrated embodiment, the manual rotary actuator 2r is removably clipped to an end portion 12a of the further moving member 12, particularly by means of elastic tabs 2c, as shown in Figure 3.

The further moving member 12 is also removably clipped to the base wall W, by its introduction in a central through hole thereof and the trapping of portions of the base wall surrounding the contour of said central through hole between elastic tabs 12b and top edge of a tubular portion 12c, as shown in Figure 3.

The base wall W (and the rest of components attached thereto) is also removably attached to the ring part 1 by means of elastic tabs f (see Figures 1 and 9) that trap an upper wall of the ring part 1 between them and an upper face of the wall W from which the elastic tabs f project upwards (according to the position illustrated in Figure 1).

Further tabs g (see Figure 9) are also provided projecting upwards from the upper face of the wall W, with the purpose of limiting a radial relative displacement between the base wall W (and the rest of components attached thereto) and the ring part 1.

As shown, among others, in Figure 1, the portable device of the first aspect of the present invention also comprises a filter F arranged between the enrichment chamber 14 and the mixture chamber 5m to filter the portion of the enriched mixture to be received by the mixture chamber 5m. For the illustrated embodiment, the filter F is a downwards and radial extension of the tubular portion 12c of the further moving member 12c.

As shown in Figure 1, for the illustrated embodiment, the portable device of the first aspect of the present invention further comprises:
- a reactive chamber 5r for containing a reactive agent, wherein the reactive chamber 5r is fluidically communicable with the enrichment chamber 14 also through a sealed, but openable, aperture 5ra (see Figure 3), for providing the reactive agent thereto through the torn first tearable seal 13; and
- an inactivating chamber 5i containing an inactivating agent, wherein the inactivating chamber 5i is fluidically communicable with the enrichment chamber 14 through a sealed, but openable, aperture 5ia (see Figure 4), for providing the inactivating agent thereto through the torn first tearable seal 13.

For a non-illustrated embodiment, only one of the reactive 5r and inactivating 5i chambers is comprised by the device.

For the illustrated embodiments, the apertures 5ra and 5ia are both sealed by other corresponding regions of the common seal 10.

As shown in Figure 1, the reactive 5r and inactivating 5i also project upwards, when the portable device is in an upright position, from respective adjacent locations of the base wall W, following the first circumferential path over the first tearable seal 13, adjacent to each other.

The reactive chamber 5r is placed adjacent to the mixture chamber 5m, and the inactivating chamber 5i is placed adjacent to the detection chamber 5d, so that during the rotation displacement of the moving member 11 about the first articulation axis A1, the region of the common seal 10 covering the aperture 5ra is first torn (by means of the same process explained above for the inlet 5ma and the aperture 5da), in order to pour a reactive agent to the enrichment chamber 14, then the one covering inlet 5ma, then the one covering aperture 5da, and finally the one covering aperture 5ia in order to pour an inactivating agent to the enrichment chamber to inactivate the contents thereof.

Vertical walls V (see Figure 1) mechanically link the chambers 5r, 5m, 5d, 5i to each other, in order to confer a higher mechanical stability thereto.

For the illustrated embodiment, different septums are provided for introducing or extracting the contents of some of the chambers, particularly, as shown in Figure 1, septums 3 for the detection 5d and inactivating 5i chambers, and septum 15 for the enrichment chamber 15.

Regarding the above mentioned mechanism intended to avoid the tearing of the region of the common tearable seal 10 sealing aperture 5da unless the one sealing inlet 5ma has already been torn, for the illustrated embodiment that mechanism comprises two tabs 2ta, 2tb (see figures 2 and 4) that project downwards from a free lower end of rotary knob 2r, and a fin B projecting radially from the outer contour of one of the chambers (in this case of inactivating chamber 5i) that before use of the device remains trapped between both tabs 2ta, 2tb, thus blocking the rotation of the rotary knob 2r and of all the components attached thereto, in both directions.

Tab 2ta is breakable, or at least foldable, by a user in order to allow the counterclockwise rotation of the rotary knob 2r and of all the components attached thereto without colliding with fin B.

The tabs 2ta, 2tb, fin B, manual rotary actuator 2, moving member 11, further moving member 12, and chambers 5r, 5m, 5d, 5i, are configured and arranged so that when the tab 2ta is broken or fold, the counterclockwise rotation can be initiated and the first region of the common seal 10 found and thus torn by the tearing portion 11a is that sealing aperture 5ra, then that sealing inlet 5ma, then that sealing aperture 5da and finally that sealing aperture 5ia.

Tab 2tb is not breakable in order to prevent a clockwise rotation of the rotary knob 2r without colliding with fin B, that would cause to invert the tearing order described above.

Therefore, by means of the correct position of all the components indicated above, the region of the common tearable seal 10 sealing aperture 5da cannot be torn if the one sealing inlet 5ma has not yet been torn, the one covering aperture 5ma cannot be torn if the one sealing aperture 5ra has not yet been torn, and the one sealing aperture 5ia cannot be torn if the one sealing aperture 5da has not yet been torn.

For an embodiment including only mixture 5m and detection 5d chambers, the same or a similar mechanism would be in charge of avoiding an incorrect order of tearing of the corresponding regions of the common tearable seal 10.

Preferably each chamber is identified by means of a corresponding indicator. For the attached Figures, those indicators are respective vertical ridges Rd: one for the reactive chamber 5r, two for the mixture chamber 5m, three for the detection chamber 5d, and four for the inactivating chamber 5i.

The present invention also relates, in a second aspect, to a method for detecting microorganisms or metabolites in a sample S, using a portable device according to any of the previous claims.

For the illustrated device, the method comprising sequentially performing an enrichment process comprising sequentially performing the following steps:
- providing the enrichment chamber 14 filled with a culture medium M sealed with a first tearable seal 13, in an upright position, as shown in Figure 5;
- depositing a sample S on the first tearable seal 13;
- tearing the first tearable seal 13, as explained above, i.e. by screwing the ring part 1 on the collecting chamber 16 up to its maximum coupling course position, including the user pressing of stop elastic member E as described above with reference to Figure 6, so that the sample S falls by gravity into the enrichment chamber, as shown in Fig. 7.
- incubating the contents (after an optional agitation thereof) of the enrichment chamber 14 to provide an enriched mixture of the culture medium M and the sample S.

The method further comprises a detection process comprising sequentially performing the following steps:
- optionally agitating the contents of the enrichment chamber 14 to homogenize the same;
- breaking or folding tab 2ta;
- counterclockwise rotate rotary knob 2r until tab 2tb reaches the indicator Rd identifying the mixture chamber 5m; at which position the region of seal 10 covering inlet 5ma has been torn as explained above;
- putting upside down the device so that a portion of the enriched mixture flows into the mixture chamber 5m from the enrichment chamber 14 through the torn first tearable seal 13, the opened inlet 5ma and tubular member 8, as explained above;
- putting the device back to the upright position, and rotate further the rotary knob 2r until tab 2tb reaches the indicator Rd identifying the detection chamber 5m; at which position the region of seal 10 covering inlet 5da has been torn so that an aliquot of the portion of the enriched mixture flows into the detection chamber 5d, as explained above; and
- detecting the presence of the microorganism or metabolite of interest in the aliquot, by means of the detection element 9; in case that element 9 is a chromatographic strip by looking thereto through the translucent region of the detection chamber 5d to look, in case of a positive detection, for a visual indicator appearing thereon that is indicative of the detection of the microorganism or metabolite of interest, after an appropriate time has passed (for example, 15 minutes).

For an embodiment for which the use of a reactive agent is needed, the method further comprises tearing the region of the seal 10 covering aperture 5ra before that of inlet 5ma, as explained above, so as to pour, while the device is in the upright position, a reactive agent contained therein into the enrichment chamber 14.

Also, for an embodiment for which after the detection process has finished, the inactivation of the enriched mixture is requested, the method of the invention comprises an inactivation process that comprises sequentially performing the following steps:
- continuing with the counterclockwise displacement of the rotary knob 2r until tab 2tb reaches the indicator Rd identifying the inactivating chamber 5i, at which position the region of seal 10 covering inlet 5ia has been torn as explained above, so as to pour, while the device is in the upright position, an inactivating reactive agent contained therein into the enrichment chamber 14;
- agitate the device to make sure that the inactivating agent reaches all the opened chambers, to homogenize its contents; and
- let the device rest for an appropriate time (such as 30 minutes) for the inactivating agent to act, in an upright position, and then in an upside down position, or vice versa;

A person skilled in the art could introduce changes and modifications in the embodiments described without departing from the scope of the invention as it is defined in the attached claims.

## Claims

1. Portable device for detecting microorganisms or metabolites in a sample (S), comprising:
- an enrichment chamber (14) for containing a culture medium (M) in a sealed manner, wherein a mouth of said enrichment chamber (14) is closed by a first tearable seal (13),
- a collecting member for collecting a sample (S) by its deposition on a supporting wall thereof;
- means for tearing said first tearable seal (13) to provide or allow the displacing of said sample (S) from said supporting wall to the interior of said enrichment chamber (14) through the torn first tearable seal (13), so that the sample (S) is enriched with said culture medium (M) for a microorganism or metabolite of interest, and an enriched mixture of said culture medium (M) and said sample (S) is provided;
- a mixture chamber (5m) fluidically communicable with the enrichment chamber (14) for receiving a portion of said enriched mixture from the enrichment chamber (14);
- a detection chamber (5d) fluidically communicable with said mixture chamber (5m) to receive at least an aliquot of said portion of the enriched mixture therefrom, said detection chamber (5d) housing a detection element (9) for detecting the presence of said microorganism or metabolite of interest in said aliquot;
**characterized in that** said mixture chamber (5m) comprises a sealed, but openable, inlet (5ma), to receive said portion of the enriched mixture from the enrichment chamber (14) flowing through said torn first tearable seal (13) and then through said inlet (5ma), once opened, and **in that** the portable device further comprises:
- an opening member for opening said sealed inlet (5ma) of the mixture chamber (5m); and
- a fluidic communication member configured and arranged for fluidically communicating the detection chamber (5d) with the mixture chamber (5m) only once the sealed inlet (5ma) of the mixture chamber (5m) is already opened.

2. The portable device according to claim 1, wherein said supporting wall and said first tearable seal (13) are the same element, so that when the first tearable seal (13) is torn by said means and the portable device is in an upright position, at which the mouth of the enrichment chamber (14) is in an upper position, the sample (S) being supported thereon is automatically displaced by falling by gravity into the enrichment chamber (13).

3. The portable device according to claim 1 or 2, wherein said opening member and said fluidic communication member are the same element, said same element being a moving member (11) configured and arranged to displace according to at least a first displacement path, to adopt a first position at which it opens the sealed inlet (5ma) of the mixture chamber (5m) and, only once said first position has been adopted, move to adopt a distinct and second position at which it fluidically communicates the detection chamber (5d) with the mixture chamber (5m).

4. The portable device according to claim 3, wherein the detection chamber (5d) is communicated with the mixture chamber (5m) through at least one fluidic channel (Ch), and comprises a sealed, but openable, aperture (5da) configured and arranged to be opened by the moving member (11) when at said distinct and second position, so that pressure within the detection chamber (5d) equals that of the mixture chamber (5m), the mixture (5m) and the detection (5d) chambers thus forming communicating vessels so that said aliquot of the portion of the enriched mixture enters into the detection chamber (5d) from the mixture chamber (5m) and passing through said at least one fluidic channel (Ch).

5. The portable device according to claim 3 or 4, wherein said inlet (5ma) of the mixture chamber (5m) is sealed by a tearable seal (10), and wherein said moving member (11) has a tearing portion (11a) configured and arranged to tear said tearable seal (10) at least when at said first position.

6. The portable device according to claim 5 when depending on claim 4, wherein said aperture (5da) of the detection chamber (5d) is sealed by a tearable seal (10), and wherein said tearing portion (11a) of the moving member (11) is configured and arranged to tear said tearable seal (10) of the aperture (5da) of the detection chamber (5d) at least when at said distinct and second position.

7. The portable device according to claim 5 or 6, wherein the moving element (11) is configured and arranged to tear, by means of the tearing portion (11a) thereof:
- the tearable seal (10) of the inlet of the mixture chamber (5m) along a plurality of positions, including said first position, that are adopted through the displacement of the moving member (11) according to at least said first displacement path; and/or
- the tearable seal (10) of the aperture of the detection chamber (5d) along a plurality of positions, including said second position, that are adopted through the displacement of the moving member (11) according to at least said first displacement path.

8. The portable device according to claim 7, wherein said moving member (11) is configured and arranged to displace also according to a second displacement path that is transversal to a plane occupied by said tearable seals (10) of the inlet (5ma) of the mixture chamber (5m) and of the aperture (5da) of the detection chamber (5d), so that during the displacement of the moving member (11) along the first displacement path the tearing portion moves according to said second displacement path towards the mixture chamber (5m) or the detection chamber (5d) when facing the corresponding tearable seal (10).

9. The portable device according to claim 8, wherein the moving member (11) is configured and arranged so that during said displacement according to said second displacement path the tearing portion:
- progressively penetrates into the mixture chamber (5m), through the corresponding tearable seal (10), increasingly until the first position is achieved along the displacement according to the first displacement path, and decreasingly therefrom, and then
- progressively penetrates into the detection chamber (5d), through the corresponding tearable seal (10), increasingly until the second position is achieved along the displacement according to the first displacement path, and decreasingly therefrom.

10. The portable device according to any of claims 3 to 9, wherein said mixture (5m) and detection (5d) chambers project upwards, when the portable device is in an upright position, from respective adjacent locations of a base wall (W), wherein said adjacent locations follow a first circumferential path over said first tearable seal (13), wherein the moving member (11) is located between the first tearable seal (13) and the mixture (5m) and detection (5d) chambers and is articulated to rotate about a first articulation axis (A1) that is orthogonal to a plane occupied by said first tearable seal (13), to displace according to said first displacement path, which is a rotation displacement path.

11. The portable device according to claim 10, comprising a manual rotary actuator (2) mechanically connected to the moving member (11), and that is accessible to a user to rotate the moving member (11) according to the first, rotating, displacement path.

12. The portable device according to claim 10 or 11 when depending on claim 9, wherein the moving member (11) is further articulated about a second articulation axis (A2) that is orthogonal to said first axis (A1) and pivots there about, to displace according to the second displacement path, which is a pivoting displacement path.

13. The portable device according to claim 12, wherein said tearing portion (11a) is located at a first end of the moving member (11), the moving member (11) having a second end (11b), opposite to said first end (11a), that is guided by means of ramps, defined by adjacent extensions (R) projecting towards the first tearable seal (13) from said base wall (W) and following a second circumferential path, to make the moving member (11) pivot about said second articulation axis (A2) when being displaced according to the first, rotating, displacement path, to provide said progressive penetration of the tearing portion (11a) into the mixture (5m) and the detection (5d) chambers.

14. The portable device according to any of the previous claims, further comprising at least one of:
- a reactive chamber (5r) for containing a reactive agent, wherein said reactive chamber (5r) is fluidically communicable with the enrichment chamber (14) through a sealed, but openable, aperture (5ra), for providing said reactive agent thereto through said torn first tearable seal (13), wherein said or another opening member is configured and arranged to open said sealed aperture (5ra) of said reactive chamber (5r) before the opening of the sealed inlet of the mixture chamber (5m); and
- an inactivating chamber (5i) containing an inactivating agent, wherein said inactivating chamber (5i) is fluidically communicable with the enrichment chamber (14) through a sealed, but openable, aperture (5ia), for providing said inactivating agent thereto through said torn first tearable seal (13), wherein said or another opening member is configured and arranged to open said sealed aperture (5ia) of said inactivating chamber (5i) only after the detection chamber (5d) has been fluidically communicated with the mixture chamber (5m).

15. A method for detecting microorganisms or metabolites in a sample (S), using a portable device according to any of the previous claims, the method comprising sequentially performing the following steps:
- providing the enrichment chamber (14) filled with a culture medium (M) in a sealed manner,
- depositing a sample (S) on said supporting wall of the collecting member;
- tearing the first tearable seal (13) and displacing the sample (S) from the supporting wall to the interior of the enrichment chamber (14) through the torn first tearable seal (13),
- incubating the contents of the enrichment chamber (14) to provide an enriched mixture of said culture medium (M) and said sample (S);
- opening the sealed inlet of the mixture chamber (5m) and providing thereto a portion of said enriched mixture from the enrichment chamber (14) through the torn first tearable seal (13) and the opened inlet;
- fluidically communicating the detection chamber (5d) with the mixture chamber (5m) to receive at least an aliquot of the portion of the enriched mixture therefrom; and
- detecting the presence of said microorganism or metabolite of interest in said aliquot, by means of said detection element (9).
